# EUROPEAN PATENT APPLICATION

(11) **EP 3 583 845 A1**
(43) Date of publication of application: **25.12.2019**
(21) Application number: 18178404.2
(22) Date of filing: 19.06.2018
(51) Int. Cl.: A01K 67/027, A61K 49/00, A61K 39/00

(54) **MAMMARY FAT PAD (MFP) TUMOR CELL INOCULATION TO AVOID TUMOR ULCERATION**

(71) Applicant: ProQinase GmbH, 79106 Freiburg (DE)
(72) Inventor: WEBER, Holger, 79115 Freiburg (DE); OBODOZIE, Cynthia, 79104 Freiburg (DE)
(74) Representative: Müller-Boré & Partner Patentanwälte PartG mbB

(57) **Abstract**

The present invention relates to a non-human mammalian animal, comprising a non-orthotopic tumor cell implant in its mammary fat pad (MFP). The present invention further relates to respective non-orthotopic tumor cell implants for use in medicine and/or oncological research, and respective uses of said implants.

## Description

The present invention relates to a non-human mammalian animal, comprising a non-orthotopic tumor cell implant in its mammary fat pad (MFP). The present invention further relates to respective non-orthotopic tumor cell implants for use in medicine and/or oncological research, and respective uses of said implants.

In 2012, around 478.000 newly-diagnosed cancer diseases were published by the *Zentrum für Krebsregisterdaten* (ZfKD), Germany. Improvements in prevention, early diagnosis, and therapy lead to a decrease in age-dependent cancer death rate in the last 10 years (around 17 % for men and 11 % for women). However, this decrease is not observed for all cancer entities. Therefore, further development of novel cancer therapies is still much needed.

Today, cancer treatment is focused mainly on three columns: surgery, chemotherapy, and target-specific therapy. For the last two columns, preclinical studies followed by clinical trials are necessary to identify new drugs. Preclinically, *in vitro* anti-tumoral effective substances are tested *in vivo* mostly in laboratory mice and rats. The substances have to be characterized regarding their anti-tumoral effects compared to controls as well as compared to already well-known medications that are already available on the market. The anti-tumoral effect is assessed by tumor volume. Only the most promising anti-tumoral effective substances will reach phase I - IV clinical trials. Thus, preclinically, tumor models must be meaning- and powerful. Tumor models which do not have that power can cause false positive or negative results. In case of false positive results, substances are further pursued without achieving marketability, wasting many resources. In case of false negative results, worthwhile substances would not be considered for further development in *in vivo* or clinical studies.

Unfortunately, in several tumor models, early development of tumor ulcerations during the study duration is observed. These ulcerated tumor-bearing mice must be euthanized according to animal ethical rules before the potential highest tumor volume can be reached.

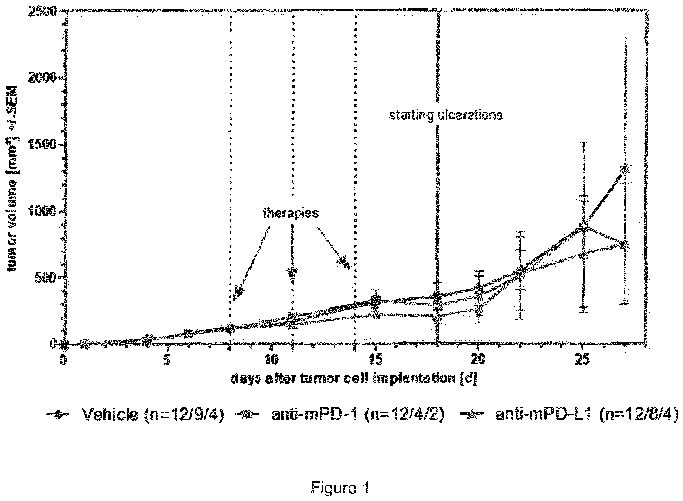

Currently, cancer research tends to focus on the development of novel cancer immunotherapies. For that reason, syngeneic tumor models will gain in importance. These tumor models are hallmarked by tumor growth in immunocompetent mice of the same mouse strain of tumor origin (syngeneic). In order to gain a broad insight into entire immunological processes, syngeneic tumor models are advantageous providing a fully developed immune system to assess novel immunotherapeutic approaches. This enables the investigation of the influence on the immune system modulated by the immune cells affecting test substances. As syngeneic tumor models are growing very fast and tumor ulceration occurs very often in an early stage of tumor growth, mice must be euthanized in an early stage. Hence, on the one hand the number of existing/remaining animals is too low to get statistical significant calculations at study end and on the other hand, study duration is too short to study the effects on the immune system, which needs time to react after test substance exposure (Figure 1).

Therefore, the technical problem underlying the present invention is the provision of means that allow for the prevention or delay of the formation of ulcerations in *in* vivo tumor models, and, thus, the increase of respective study durations.

The solution to the above technical problem is achieved by the embodiments characterized in the claims.

In particular, in a first aspect, the present invention relates to a non-human mammalian animal, comprising a non-orthotopic tumor cell implant in its mammary fat pad (MFP).

In related aspects, the present invention relates to a non-orthotopic tumor cell implant for use in medicine or for use in oncological research, wherein the non-orthotopic tumor cell implant is located in the MFP of a non-human mammalian animal.

In a further aspect, the present invention relates to the use of a non-orthotopic tumor cell implant in oncological research, wherein the non-orthotopic tumor cell implant is located in the MFP of a non-human mammalian animal.

In all of these aspect, the non-human mammalian animal is preferably a mouse (*Mus musculus*) or a rat (*Rattus norvegicus*), wherein a mouse is particularly preferred. In case the animal is a mouse, the mouse can be derived from any suitable mouse strain known in the art, including major mouse strains known in the art and their respective substrains, including genetically modified descendants, and is e.g. selected from the group consisting of BALB/c, C57BL/6, C57BL/6 albino, DBA, CBA, C3H, A, NMRI, NMRI nude, athymic nude, BALB/c nude, CB17SCID, hairless SCID, SCID beige, and nod SCID. In case the animal is a rat, the rat can be derived from any suitable rat strain known in the art, including major rat strains known in the art and their respective substrains, including genetically modified descendants, and is e.g. selected from the group consisting of Wistar, COP, Fischer, and BDIX.

The term "mammary fat pad (MFP)" as used herein relates to fat tissue that surrounds the ductal system in the mammary glands, as well as the mammary glands itself, of a female mammal. In this context, methods for implanting a tumor cell implant into the MFP of a non-human mammal are not particularly limited and are known in the art.

The tumor cell implant defined in the above aspects is an implant of a number of tumor cells in the MFP of the non-human mammalian animal. As used herein, the term "non-orthotopic" in the context of a tumor cell implant indicates that the tumor cells are not derived from cells of the MFP, e.g. as determined by pathohistological examinations, i.e., the tumor cells are not breast tumor cells. As defined herein, non-orthotopic tumor cells are also tumor cells established from the MFP, which originate from a primary tumor of another entity than breast. Preferably, the tumor cell implant is derived from a tumor cell line, selected from the group consisting of LL-2 (Lewis Lung), Ct26wt, MC38-CEA, AB12, B16, Clone M3, Hepa1-6, RENCA, C6, P388, Madison M109, L5178y, J558L, Meth A, M5076, TRAMP-C2, A20, C1498, LA-4, C51, L1210, MBT-2, MPC-11, TC-1, Colon26, WEHI-164, GL261, H22, KLN205, E.G7-OVA, EL4, LS178, P815, J558, P3X63Ag8U.1, N1E-115, Neuro-2a, Pan02, Panc-02, RM-1, AY27, GS-9L, GV1A1, R3327, and NBT-II, including their derivatives and transduced descendants, and human tumor cell lines A2058, A375, A431, A549, Alexander, AsPC1, BxPC-3, C33A, Calu-6, Colo-205, DU 145, H460, HCT-116, HeLa, HL-60, Hs746T, HT29, Hutu80, KARPAS-299, LN-229, LNCaP, LoVo, LS 174T, MiA-PaCa2, MKN-1, MKN-45, MOLM-13, MV4-11, NCI-H69, OVCAR-3, PC-3, RKO, SJSA-1, SK-N-MC, SKOV-3, SW620, and U-87 MG, including their derivatives and transduced descendants.

In specific embodiments, the tumor cell line is a syngeneic tumor cell line, i.e., it is derived from the same species and strain as the non-human mammalian animal.

The term "comprise(s)/comprising" as used herein is expressly intended to encompass the terms "consist(s)/consisting essentially of" and "consist(s)/consisting of".

The present invention is based on the chance observation for the orthotopic implanted 4T1 tumor model as well as the EMT6 tumor model (both breast cancer tumor models) that tumor ulcerations appeared with much lower frequency and at a later time point in comparison to subcutaneously implanted cells. This led to the idea to implant other tumor cells than breast cancer cells into the mammary fat pad (i.ma.) instead of subcutaneously. Subcutaneous implanted tumors serve as a standard model in oncology. However, in view of the findings in the present invention, implantation into mammary fat pad might serve as a superior implantation site. Both implantation methods are heterotopic and can be useful as exemplary tumor models for preclinical drug testing.

The figures show:
Figure 1:
   Murine EMT-6 breast tumor cells were subcutaneously implanted into immune competent BALB/c mice. After randomization on day 8 animals were treated three times with Vehicle, anti-PD-1 and anti PD-L1, respectively. On day 15 the first antitumoral effects of anti-PD-L1 treatment was observed, three days later the first tumor ulcerations occurred, leading to reduced animals numbers per group (in brackets animal number n on day 8/day 18/day 27).
Figure 2:
   Ct26wt colon cancer cells were implanted into the mammary fat pad. Tumor growth was monitored over time (top) and a survival Kaplan Meier curve was created indicating animal death/euthanasia during the study (bottom).
Figure 3:
   LL2 lung cancer cells were implanted into the mammary fat pad. Tumor growth was monitored over time (top) and a survival Kaplan Meier curve was created indicating animal death/euthanasia during the study (bottom).
Figure 4:
   MC38-CEA colon cancer cells were implanted into the mammary fat pad. Tumor growth was monitored over time (top) and a survival Kaplan Meier curve was created indicating animal death/euthanasia during the study (bottom).

The present invention will be further illustrated by the following example without being limited thereto.

### Example

To overcome the limitations of subcutaneous tumor models regarding early development of ulcerations, which leads to premature end of the study, a non-subcutaneous tumor inoculation site for tumor cell lines other than breast tumor cells, *i.e*., the mammary fat pad, has been evaluated.

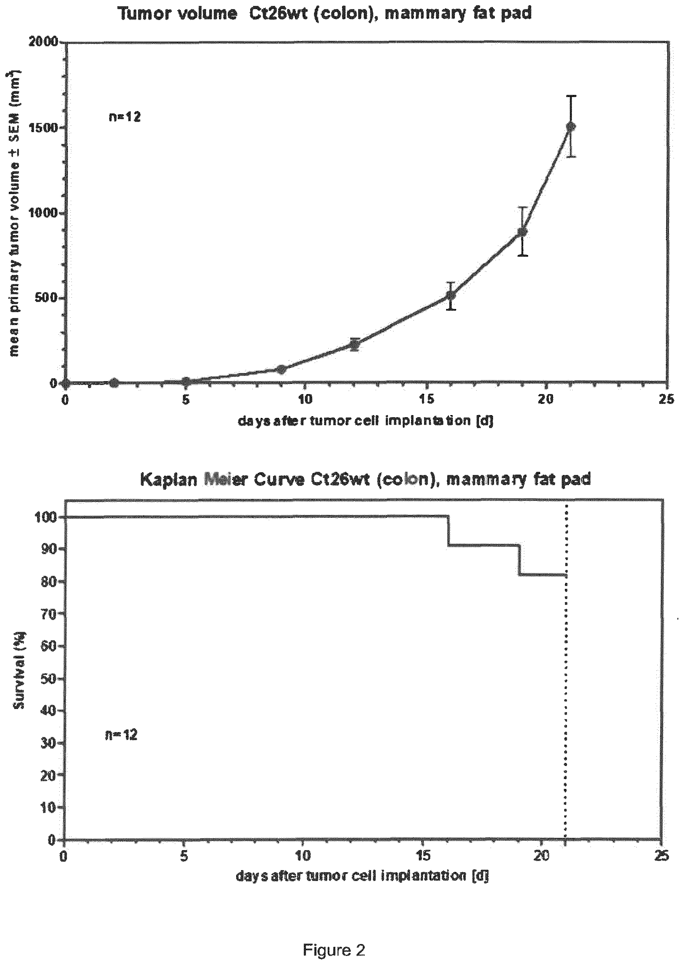

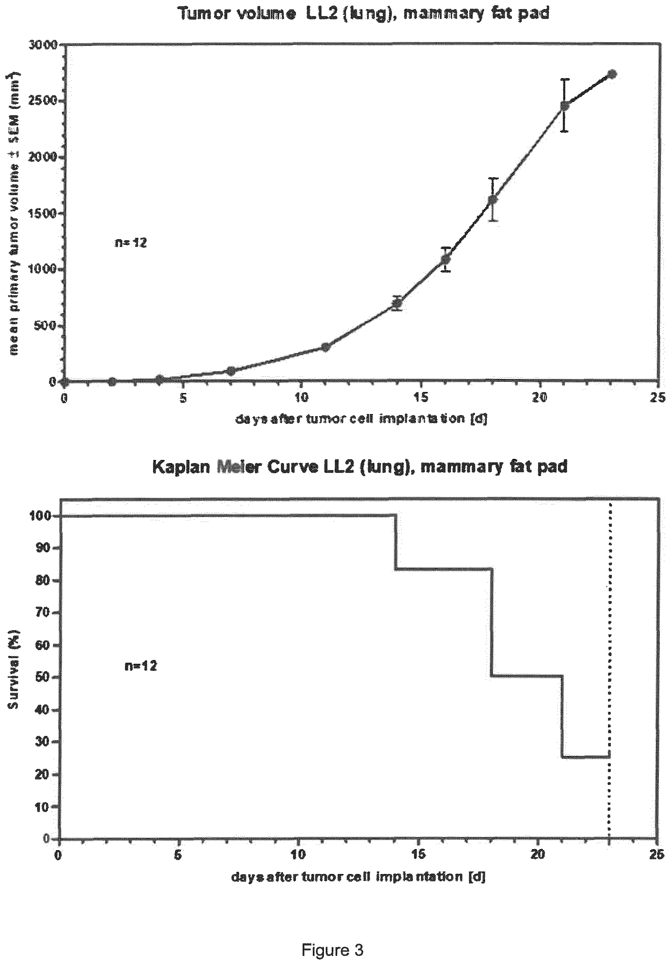

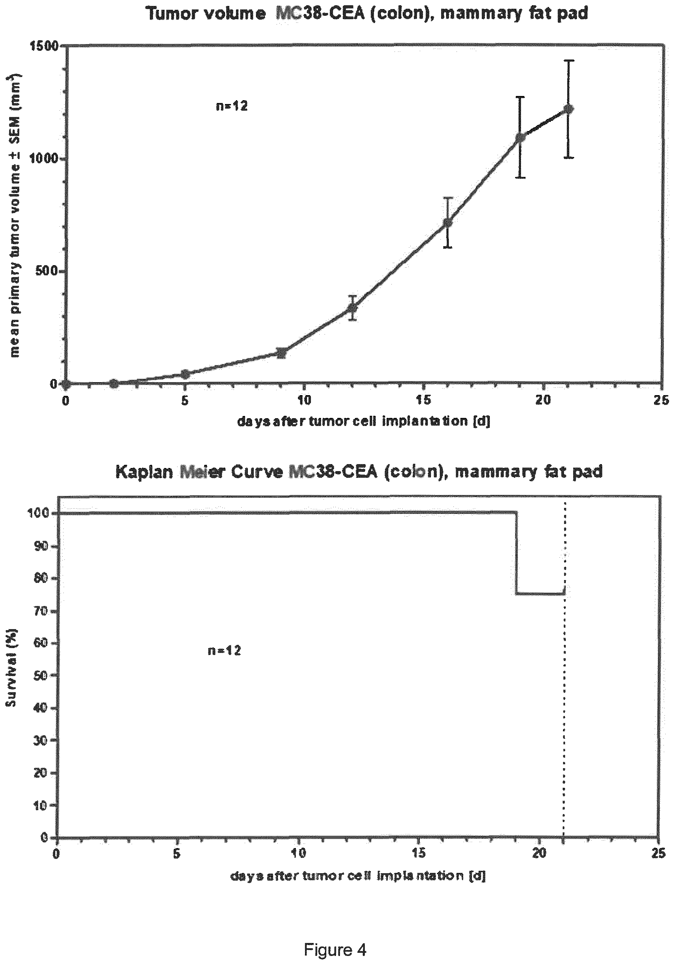

In summary, tumor ulceration could successfully be prevented by using the mammary fat pad as alternate implantation site, and, therefore, final tumor volume could be increased. Thus, study duration is extended, depending on the tumor model (Table 1) as in i.ma. implantation, the study end criteria is mainly tumor size (> 2 cm) and not tumor ulceration. Three study examples for MFP implantations are shown in Figures 2-4: Ct26wt (colon), LL2 (lung) and MC38-CEA (colon).

**Table 1: Summary of the differences in study duration, tumor growth, remaining animals at study end, as well as tumor ulcerations in tumor cells implanted either subcutaneously or into the mammary fat pad.**

| **Cell line** | **Implantation site** | **Study duration (days)** | **Final tumor volume (mm³)** | **Ratio final tumor volume (s.c./i.ma.)** | **Remaining animals at study end** | **Ratio animals at study end (s.c./i.ma.)** | **Tumor ulcerations** | **Ratio tumor ulceration (s.c./i.ma.)** |
|---|---|---|---|---|---|---|---|---|
| **Ct26wt** | s.c. | 19 | 500 | **1/3** | 33% | **1/2.5** | 8 | **8/1** |
| | i.ma. | 21 | 1500 | | 83% | | 1 | |
| **LL2** | s.c. | 14 | 500 | **1/5.4** | 0% | **undefined** | 12 | **3/1** |
| | i.ma. | 23 | 2700 | | 25% | | 4 | |
| **MC38-CEA** | s.c. | 19 | 300 | **1/4.6** | 41.6% | **1/1.8** | 7 | **7/0** |
| | i.ma. | 21 | 1400 | | 75% | | 0 | |

## Claims

1. A non-human mammalian animal, comprising a non-orthotopic tumor cell implant in its mammary fat pad (MFP).

2. A non-orthotopic tumor cell implant for use in medicine, wherein the tumor cell implant is located in the mammary fat pad (MFP) of a non-human mammalian animal.

3. A non-orthotopic tumor cell implant for use in oncological research, wherein the tumor cell implant is located in the mammary fat pad (MFP) of a non-human mammalian animal.

4. Use of a non-orthotopic tumor cell implant in oncological research, wherein the tumor cell implant is located in the mammary fat pad (MFP) of a non-human mammalian animal.

5. The non-human mammalian animal, the non-orthotopic tumor cell implant for use, or the use according to anyone of claims 1 to 4, wherein the animal is a mouse (*Mus musculus*) or a rat (*Rattus norvegicus*)*.*

6. The non-human mammalian animal, the non-orthotopic tumor cell implant for use, or the use according to claim 5, wherein the animal is a mouse (*Mus musculus*)*.*

7. The non-human mammalian animal, the non-orthotopic tumor cell implant for use, or the use according to claim 6, wherein the mouse is derived from a mouse strain, selected from the group, consisting of BALB/c, C57BL/6, C57BL/6 albino, DBA, CBA, C3H, A, NMRI, NMRI nude, athymic nude, BALB/c nude, CB17SCID, hairless SCID, SCID beige, and nod SCID.

8. The non-human mammalian animal, the non-orthotopic tumor cell implant for use, or the use according to claim 5, wherein the animal is a rat (*Rattus norvegicus*)*.*

9. The non-human mammalian animal, the non-orthotopic tumor cell implant for use, or the use according to claim 8, wherein the rat is derived from a rat strain, selected from the group, consisting of Wistar, COP, Fischer, and BDIX.

10. The non-human mammalian animal, the non-orthotopic tumor cell implant for use, or the use according to any one of claims 1 to 9, wherein the tumor cell implant is derived from a tumor cell line, selected from the group consisting of LL-2, Ct26wt, MC38-CEA, AB12, B16, Clone M3, Hepa1-6, RENCA, C6, P388, Madison M109, L5178y, J558L, Meth A, M5076, TRAMP-C2, A20, C1498, LA-4, C51, L1210, MBT-2, MPC-11, TC-1, Colon26, WEHI-164, GL261, H22, KLN205, E.G7-OVA, EL4, LS178, P815, J558, P3X63Ag8U.1, N1E-115, Neuro-2a, Pan02, Panc-02, RM-1, AY27, GS-9L, GV1A1, R3327, and NBT-II, A2058, A375, A431, A549, Alexander, AsPC1, BxPC-3, C33A, Calu-6, Colo-205, DU 145, H460, HCT-116, HeLa, HL-60, Hs746T, HT29, Hutu80, KARPAS-299, LN-229, LNCaP, LoVo, LS 174T, MiA-PaCa2, MKN-1, MKN-45, MOLM-13, MV4-11, NCI-H69, OVCAR-3, PC-3, RKO, SJSA-1, SK-N-MC, SKOV-3, SW620, and U-87 MG.

11. The non-human mammalian animal, the non-orthotopic tumor cell implant for use, or the use according to any one of claims 1 to 10, wherein the tumor cell implant is a syngeneic tumor cell implant.
